# EUROPEAN PATENT SPECIFICATION

(11) **EP 1 527 737 B1**
(45) Date of publication and mention of the grant of the patent: **10.01.2007**
(21) Application number: 04256698.4
(22) Date of filing: 29.10.2004
(51) Int. Cl.: A61B 5/15

(54) **Lancing device with trigger mechanism for penetration depth control**
Lanzette mit Auslösevorrichtung zur Steuerung der Eindringtiefe
Lancette avec mecanisme déclencheur pour la commande de la profondeur de pénétration

(30) Priority: 31.10.2003 US 698775
(43) Date of publication of application: 04.05.2005
(73) Proprietor: LifeScan, Inc., Milpitas, CA 95035 (US)
(72) Inventor: Allen, John J., Mendota Heights, MN 55118 (US)
(74) Representative: Mercer, Christopher Paul

(56) References cited:
- WO-A-03/007819
- US-A1- 2001 027 327

## Description

### BACKGROUND OF THE INVENTION

### 1. Field of the Invention

The present invention relates, in general, to lancing devices and, in particular, to lancing devices with penetration depth control and associated methods of use.

### 2. Description of the Related Art

Conventional lancing devices generally have a rigid housing and a lancet that can be armed and launched so as to briefly protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A biological fluid sample (e.g., a whole blood sample) can then be expressed from the penetrated target site for collection and analysis. Conventional lancing devices are described in U.S. Patent No. 5,730,753 to Morita, U.S. Patent No. 6,045,567 to Taylor et al. and U.S. Patent No. 6,071,250 to Douglas et al.

Lancing devices often include a cap that engages the target site. Such a cap has an aperture (i.e., opening), through which the lancet protrudes during use. Typically, a distal end of the cap will be placed in contact with the target site during use. The profile of the distal end of the cap can be adapted for contact with predetermined target sites, such as fingers, earlobes, forearms and the abdomen.

When a cap is contacted with a target site, pressure is usually applied to the target site prior to launch of the lancet. This pressure urges the cap against the target site and creates a target site bulge within the opening of the cap. The lancet is then launched to penetrate the target site bulge.

When pressure is applied by such a cap against a target site, however, the height of the resultant target site bulge can vary greatly depending on the dimensions of the cap's opening, the magnitude of applied pressure and various physical properties (e.g., elasticity) of the target site. Such variability in target site bulge height causes the penetration depth of the lancet into the target site bulge to vary as well. Thus, a lancet can potentially penetrate too deeply in some circumstances and not deeply enough, or at all, in other circumstances. Still needed in the field, therefore, is a lancing device and associated method that provide for a controlled and consistent penetration depth.

### SUMMARY OF THE INVENTION

Lancing devices and associated methods according to embodiments of the present invention provide for a controlled and consistent penetration depth. A lancing device according to an exemplary embodiment of the present invention includes a housing, a lancing mechanism operatively attached to the housing, a pressure tip (e.g., a pressure ring) and a trigger mechanism. The pressure tip is moveably attached to the housing and is configured to engage a target site and create a target site bulge.

The trigger mechanism is configured for detecting a target site bulge of a predetermined height and, thereafter, triggering an immobilization of the pressure tip with respect to the housing. The immobilization of the pressure tip prevents subsequent change in the target site bulge location relative to the housing. Since the location of the target site bulge relative to the housing is controlled by the trigger mechanism, via immobilization of the pressure tip, penetration depth remains consistent upon each use of the lancing device according to the present invention.

A method for lancing a target site according to an exemplary embodiment of the present invention includes first providing a lancing device (according to the present invention as described herein), followed by contacting a pressure tip of the lancing device with the target site. Next, the pressure tip is urged towards the target site, thereby creating a target site bulge that is detected by a trigger mechanism of the lancing device. The trigger mechanism thereafter triggers an immobilization of the pressure tip with respect to the housing. Subsequently, the target site bulge is lanced with a lancet mechanism of the lancing device.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIG. 1 is a perspective exploded view of a lancing device according to an exemplary embodiment of the present invention;
FIG. 2 is a perspective view of distal portion of the lancing device of FIG. 1;
FIG. 3A is a simplified, schematic, cross-sectional view of a distal portion of the lancing device of FIG. 1 during initial contact with a target site;
FIG. 3B is a simplified, schematic, cross-sectional view of a distal portion of the lancing device of FIG. 1 during formation of a target site bulge;
FIG. 3C is a simplified, schematic, cross-sectional view of a portion of the lancing device of FIG. 1 depicting movement of the pressure ring relative to the housing of the lancing device;
FIG. 3D is a simplified, schematic, cross-sectional view of a distal portion of the lancing device of FIG. I depicting the trigger mechanism immobilizing the pressure ring with respect to the housing;
FIG. 3E is a simplified, schematic, cross-sectional view of a distal portion of the lancing device of FIG. 1 depicting the lancing of the target site bulge; and
FIG. 4 a flow diagram illustrating a sequence of steps for lancing a target site according to the present invention.

### DETAILED DESCRIPTION OF THE INVENTION

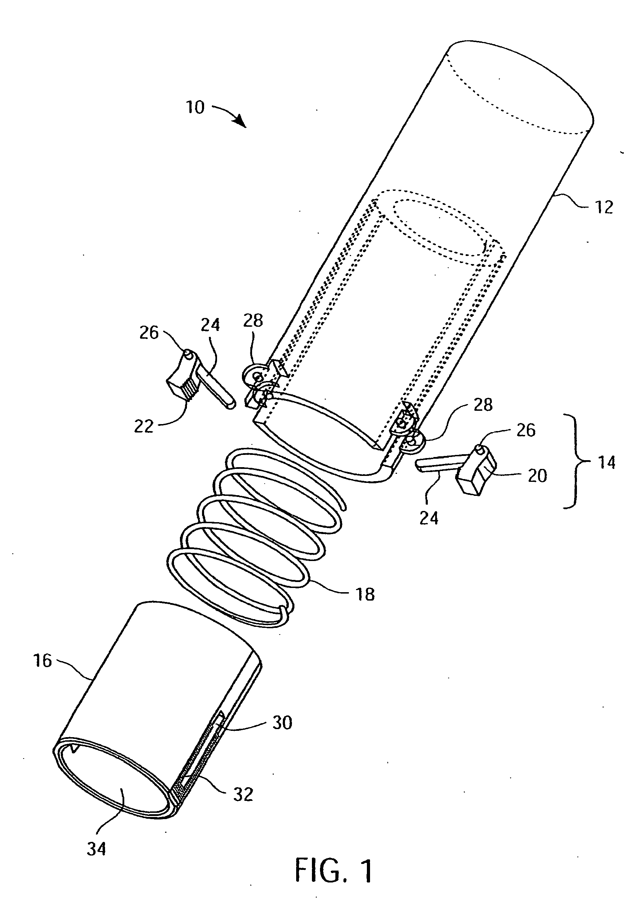

FIG. 1 shows a perspective exploded view of an exemplary embodiment of a lancing device 10 according to the present invention. Lancing device 10 includes a housing 12, trigger mechanism 14, pressure tip 16, bias spring 18 and a suitable lancing mechanism (not shown). The lancing mechanism is operatively attached to the housing and can include a launch spring, lancet carriage, lancet holder and lancet. Once apprised of the present disclosure, one of ordinary skill in the art will recognize suitable lancing mechanisms. Exemplary lancing mechanisms that are suitable for use are described in U.S. Patent Nos. 6,045,567 and 6,197,040.

Trigger mechanism 14 includes two locking pawls 20 (with pawl ratchet teeth 22), pawl trigger arms 24 and axes 26. Trigger mechanism 14 is configured for detecting a target site bulge of a predetermined height and, thereafter, triggering an immobilization (locking) of pressure tip 16 with respect to housing 12, thereby preventing any subsequent change in a target site bulge location relative to the housing.. Typical, but non-limiting, target site bulge heights that can be detected by the trigger mechanism are in the range of 0 mm to 5 mm. One skilled in the art will recognize, however, that target site bulge heights can exceed 5 mm depending on, for example, the dimensions of the pressure tip. Locking pawls 20 can be formed of any suitable rigid material including, but not limited to, acrylonitrile butadiene styrene plastic material, injection moldable plastic material, polystyrene material or metallic material. If desired, trigger mechanism 14 can also be configured, via mechanical, electrical and/or other suitable means known to one skilled in the art, to initiate lancing by the lancing mechanism once the pressure tip has been immobilized.

Once apprised of the present disclosure, one skilled in the art will recognize that the trigger mechanism of lancing devices according to the present invention can take forms other than the locking pawls, trigger arms and axes of trigger mechanism 14. For example, the trigger mechanism can include a frictional clutch or electromagnetic locking mechanism, instead of pawl ratchet teeth, adapted for immobilizing the pressure ring. Furthermore, the trigger mechanism of lancing devices according to the present invention could include an electrical or optical relay switch configured to detect a target site bulge of predetermined height. Such an optical relay switch can include, for example, a light emitter and a light detector mounted on the housing such that a target site bulge of predetermined height can be optically detected. Such detection can occur, for example, by the target site bulge interrupting an optical communication pathway between the light emitter and light detector or by the target site bulge acting as a reflector that creates an optical communication pathway between the light emitter and light detector. In addition, such an optical relay switch can be operatively linked to an electromechanical lock that immobilizes the pressure tip once a target site bulge of predetermined height has been detected by the optical relay switch.

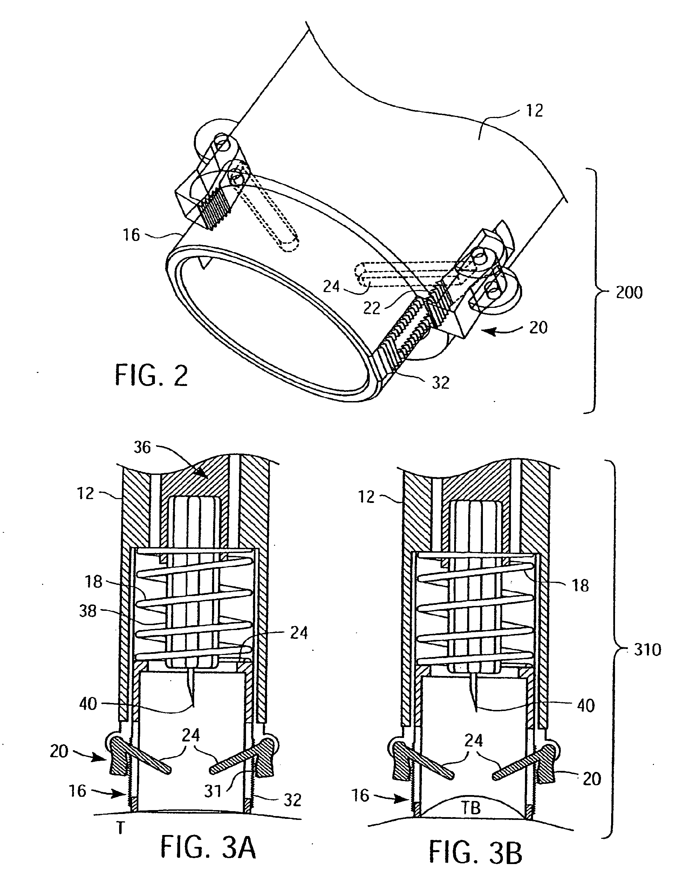

Housing 12 includes pawl attachment fixtures 28. In addition, pressure tip 16 is moveably attached to housing 12 and includes through-slots 30, pressure tip ratchet teeth 32 (configured to engage with pawl ratchet teeth 22 as described below), and opening 34. Pressure tip 16 is configured for engaging a target site (e.g., a dermal tissue target site) and creating a target site bulge (not shown in FIG. 1). In the embodiment of FIG. 1, pressure tip 16 fits at least partially within housing 12. Through-slots 30 of pressure tip 16 are configured to provide for pawl trigger arms 24 to project into opening 34 of pressure tip 16 (as illustrated in detail in FIGs. 2 and 3A through 3E below). In addition, housing 12 can include projections (not shown in the figures) or other suitable means for preventing pressure tip 16 from being inadvertently displaced entirely from within housing 12. Exemplary pressure tips that are suitable for use are described in U.S. Patent No. 6,203,504 and U.S. Patent Application Publication No. 2002/0016606.

Opening 34 may be, but is not limited to, a circular shape opening, square shape opening, triangular shape opening, C-shape opening, U-shape opening, hexagonal shape opening and an octagonal shape opening. In addition, the surface of pressure tip 16 may be, but is not limited to, smooth, rounded edges or a contoured profile as described in U.S. Patent Application Publication No. 2002/0016606. Pressure tip 16 can be formed of, for example, a rigid or a relatively resiliently deformable material, including, but not limited, to elastomeric materials, polymeric materials, polyurethane materials, latex materials, silicone materials and any combinations thereof.

Bias spring 18 is configured to apply a pre-load force in the range of about 3 N to 13 N against pressure tip 16 and preferably applies a pre-load force of in the range of 9 N to 10 N against pressure tip 16. The pre-load force of bias spring 18 serves to provide for a predetermined minimum force (i.e., a minimum force equal to the pre-load force of the bias spring) to be applied to a target site before penetration thereof. Such a predetermined minimum force has proven beneficial for increasing the volume of sample expressed from a target site. As bias spring 18 is compressed during use, the force applied by the bias spring can increase. A typical, but non-limiting, increase in the applied force is less than 5% of the pre-load force.

FIG. 2 depicts a distal portion 200 of lancing device 10 in an assembled state, with dashed lines indicating elements that are hidden from view. Prior to use, a portion of pressure tip 16 is retained in housing 12 and a portion 210 of pressure tip 16 extends from the end of housing 12. This retained portion of pressure tip 16 serves to facilitate longitudinal movement of pressure tip 16 along a straight line within housing 12. In the embodiment of FIG. 2, the portion of pressure tip 16 that extends from housing 12 extends a distance in the range of about 6 mm to 12 mm. This extended portion of pressure tip 16 serves to provide a distance for the pressure tip to move within the housing while avoiding undesirable interference between the target site and any components of the lancing device. In addition, FIG. 2 depicts the manner in which trigger mechanism 14 is pivotally attached to housing 12 via axes 26 and pawl attachment fixtures 28.

Locking pawls 20 are angularly biased by light springs (not shown) such that pawl ratchet teeth 22 and pressure tip ratchet teeth 32 are disengaged prior to use of the lancing device, as shown in FIG. 2. Such light springs are selected and configured to apply a force of, for example, less than 1 N (for example, 0.2 N or less) in order to angularly bias locking pawls 20.

FIGs. 3A through 3E depict lancing device 10 during various stages of a process for lancing a target site (T). As noted above, lancing device 10 includes a suitable lancing mechanism. FIGs. 3A through 3E depict a lance holder 36 and attached lancet 38 (with lancet tip 40) of such a suitable lancing mechanism.

Lancet 38 can be, for example, any suitable disposable lancet known to one skilled in the art. Those skilled in the art will also recognize that lancet 38 can be replaced with an integrated lance-strip device, such as that disclosed in U.S. Patent Application 10/143,399, International Patent Application No. PCT/US01/07169, International Application No. PCT/GB01/05634 (published as WO 02/49507 on June 27, 2002), and International Patent Application No. PCT/GB02/03772.

FIG. 3A is a schematic, cross-sectional view of a distal portion 310 of lancing device 10 during initial contact with a target site (T), such as a dermal tissue target site. Such an initial contact can, for example, occur with a minimal amount of force (e.g., a force in the range of about 3N to 13 N). Under such a minimal amount of force, target site T has not developed a significant target site bulge.

FIG. 3B is a schematic, cross-sectional view of distal portion 310 during the initial formation of a target site bulge (TB). As lancing device 10 is pressed against the target site, pressure tip 16 engages the target site and creates a target site bulge TB within opening 34. At the stage depicted in FIG. 3B (i.e., the initial formation of a target site bulge), the amount of force applied by lancing device 10 to the target site approaches, but does not exceed, the pre-load force of bias spring 18. Thus, pressure ring 16 has not moved relative to housing 12.

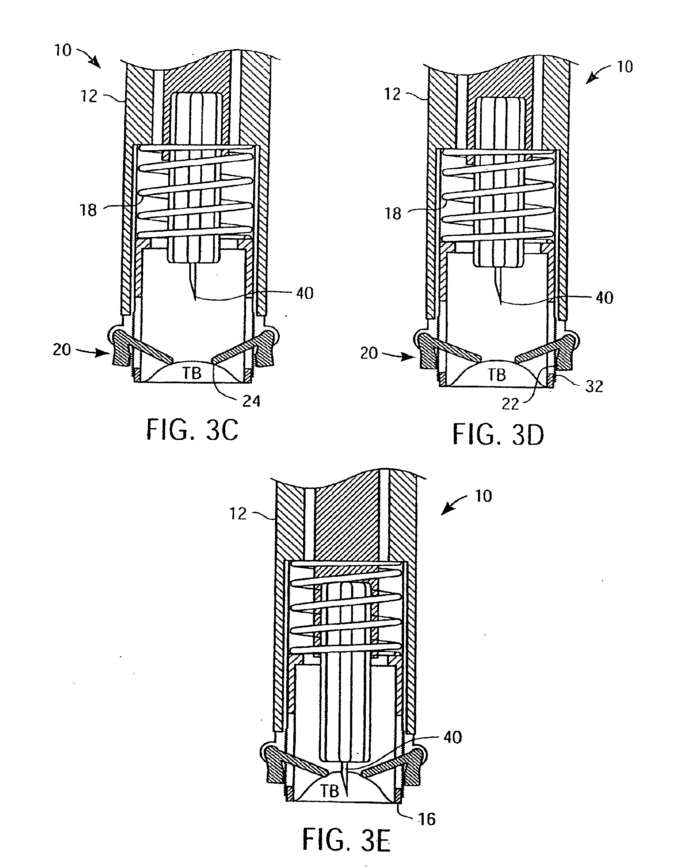

FIG. 3C is a schematic, cross-sectional view of portion 310 depicting movement of the pressure tip relative to the housing of the lancing device. As the amount of force applied to the target site is increased, the applied force exceeds the pre-load force of bias spring 18 and pressure tip 16 moves relative to housing 12 until the target site bulge contacts pawl trigger arms 24. Yet further application of force causes locking pawls 20 to rotate and immobilize (i.e., lock) pressure tip 16 in place with respect to housing 12 via paw ratchet teeth 22 and pressure tip ratchet teeth 32, as illustrated in FIG. 3D. This immobilization prevents further change in the location of the target site bulge relative to the housing. The prevention of further change can be facilitated by, for example, use of a bias spring of sufficient pre-load force that application of additional force following immobilization of the pressure tip does not appreciably increase the height of the target site bulge. In other words, the bias spring can be chosen such that the target site bulge reaches its maximum height under the pre-load force of the bias spring. Furthermore, in the event that insufficient force is applied to the target site for the target site bulge to contact pawl trigger arms 24, it would be desirable for the lancing mechanism to be prevented from lancing the target site. This can be achieved by, for example, operatively linking the trigger mechanism to the lancing mechanism.

FIG. 3E is a schematic, cross-sectional view of distal portion 310 of the lancing device of FIG. 1 depicting the lancing of the target site bulge by lancet tip 40. The penetration depth of lancet tip 40 into the target site bulge remains constant across different bulge heights since the bulge height is constrained by the locked pressure tip such that the position of the upper surface of the target site bulge is unchanged relative to housing 12. The skin bulge should be shown making contact with the pawl arms as in FIGs. 3C and 3D.

Since users may have a preferred penetration depth (due to variables such as target site quality or thickness), the penetration depth may be adjustable in the range of 0.3 to 2 mm, and preferably 0.5 to 1.0 mm by techniques that are known to those of skill in the art (see, for example, European Patent Application [filed October 19, 2003, entitled "Lancing Device with a Floating Probe for Control of Penetration Depth", identified by Attorney's Docket No. P039099EP. However, in lancing devices according to the present invention, the target site bulge is consistently positioned within housing 12 upon each use of the lancing device. Since the position of the target site bulge is controlled by the trigger mechanism, via immobilization of the pressure tip, penetration depth remains constant and is, therefore, controlled.

If desired after lancing, an over-travel spring (not shown) can be employed to withdraw (retract) lancet 38 by several millimeters, for example, to rest at a location near or just below the surface of the target site bulge at a depth in the range of approximately 0.05 to 0.25 mm. This facilitates *in-situ* testing of a fluid sample by means of fluid collection device (such as a test strip) that is introduced at the target site after a lancet has been withdrawn. Such a withdrawal of a lancet is described in Provisional U.S. Patent Application No. 60/422,228. Following use of the lancing device and removal of the lancing device from the target site, the light springs serve to bias the locking pawls such that the ratchet teeth are disengaged and the immobilization of the pressure tip released.

As will be appreciated by those skilled in the art, lancet devices according to the present invention are advantageous in that they greatly facilitate reproducible production of fluid sample (e.g., a blood sample) at a target site due to the consistency of penetration depth.

Another advantage of lancing devices according to the present invention is that a user is not required to make an adjustment to optimize the position of the target site bulge since the pressure tip and trigger mechanism operate to automatically position a target site bulge within the housing for optimal lancing. Thus, fewer steps are required to obtain a suitable fluid sample and the possibility of having to repeatedly lance , or of wasting an analyte test strip due to insufficient sample, is reduced.

Yet another advantage of lancing devices according to embodiments of the present invention is that lancing mechanism can be operatively decoupled from the pressure tip and trigger mechanism. Therefore, any launching spring that may be included in the lancing mechanism does not apply any inappropriate force against the target site bulge via the pressure tip.

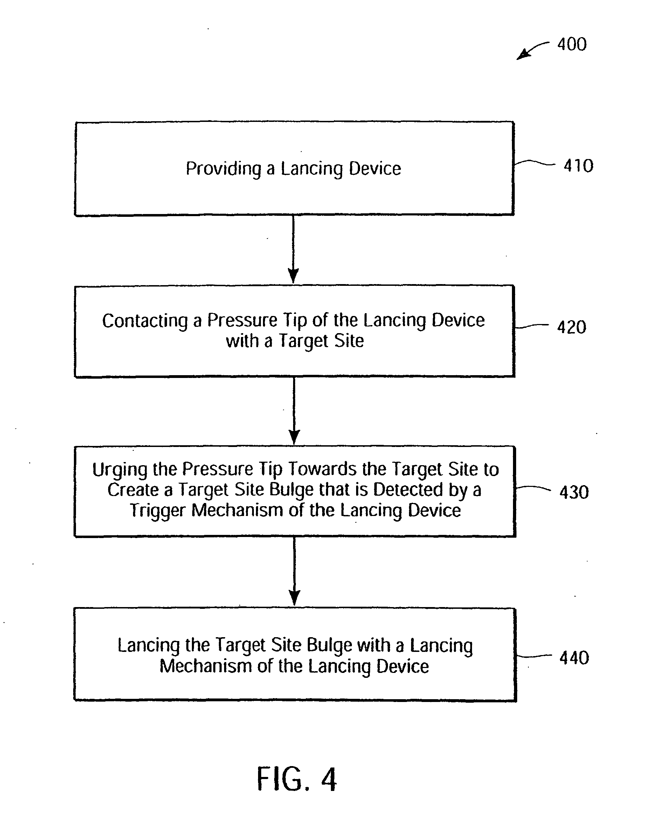

Referring to FIG. 4, a method 400 for lancing a target site includes providing a lancing device according to the present invention as described above, as set forth in step 410. The lancing device includes a housing, a lancing mechanism operatively attached to the housing, a pressure tip and a trigger mechanism. The pressure tip of the lancing device is moveably attached to the housing and is configured to engage a target site and create a target site bulge. Furthermore, the trigger mechanism is configured for detecting a target site bulge of a predetermined height and, thereafter, triggering an immobilization of the pressure tip with respect to the housing.

Next, at step 420, the pressure tip of the lancing device is contacted with the target site (e.g., a dermal tissue target site of a finger, forearm, abdomen or earlobe). The pressure tip is then urged towards the target site, thereby creating target site bulge that is detected by the trigger mechanism and triggering an immobilization of the pressure tip with respect to the housing, as set forth in step 430.

Next, the target site bulge is lanced with the lancing mechanism (for example, the target site can be lanced by launching a lancet tip included in the lancing mechanism), as set forth in step 440. One skilled in the art will recognize that steps 410,420,430 and 440 have been effectively illustrated by FIGs. 2 through 3E above.

It should be understood that various alternatives to the embodiments of the invention described herein may be employed in practicing the invention. It is intended that the following claims define the scope of the invention and that methods and structures within the scope of these claims be covered thereby.

## Claims

1. A lancing device comprising:
a housing;
a lancing mechanism operatively attached to the housing,
a pressure tip for engaging a target site and creating a target site bulge, the pressure tip moveably attached to the housing; and **characterized by**
a trigger mechanism for detecting a target site bulge of a predetermined height and, thereafter, triggering an immobilization of the pressure tip with respect to the housing, thereby preventing subsequent change in target site bulge location relative to said housing

2. The lancing device of claim 1, wherein the lancing device further includes:
a bias spring for applying a pre-load force against the pressure tip.

3. The lancing device of claim 2, wherein the bias spring is configured to apply a pre-load force in the range of 3N to 13 N against the pressure tip.

4. The lancing device of claim 2, wherein the bias spring is configured to apply a pre-load force in the range of 9N to 10 N against the pressure tip.

5. The lancing device of claim 1, wherein the trigger mechanism includes at least one locking pawl and at least one pawl trigger arm.

6. The lancing device of claim 5, wherein the locking pawl includes pawl ratchet teeth and wherein the pressure tip includes pressure tip ratchet teeth.

7. The lancing device of claim 1, wherein the trigger mechanism includes a frictional clutch for immobilizing the pressure tip.

8. The lancing device of claim 1, wherein the trigger mechanism includes an optical relay switch configured to detect a target site bulge of a predetermined height.

9. The lancing device of claim 1, wherein the trigger mechanism includes an electrical relay switch.

10. The lancing device of claim 1, wherein the trigger mechanism is configured to initiate lancing by the lancing mechanism once the pressure tip has been immobilized.

## Patentansprüche

1. Lanzettenvorrichtung, welche umfaßt:
ein Gehäuse;
einen Lanzettenmechanismus, welcher mit dem Gehäuse funktionell verbunden ist,
ein Druckende zum Angreifen an eine Zielstelle und Erzeugen einer Zielstellenwulstung, wobei das Druckende an dem Gehäuse beweglich angebracht ist;
und welche **gekennzeichnet ist durch**
einen Auslösemechanismus zum Detektieren einer Zielstellenwulstung einer vorgegebenen Höhe und zum nachfolgenden Auslösen eines Feststellens des Druckendes bezüglich des Gehäuses, wodurch eine spätere Änderung der Lage der Zielstellenwulstung relativ zum Gehäuse verhindert wird.

2. Lanzettenvorrichtung nach Anspruch 1, wobei die Lanzettenvorrichtung femer eine Vorspannfeder zum Ausüben einer Vorspannkraft gegen das Druckende umfaßt.

3. Lanzettenvorrichtung nach Anspruch 2, wobei die Vorspannfeder dazu eingerichtet ist, eine Vorspannkraft im Bereich von 3N bis 13N gegen das Druckende auszuüben.

4. Lanzettenvorrichtung nach Anspruch 2, wobei die Vorspannfeder dazu eingerichtet ist, eine Vorspannkraft im Bereich von 9N bis 10N gegen das Druckende auszuüben.

5. Lanzettenvorrichtung nach Anspruch 1, wobei der Auslösemechanismus wenigstens eine Sperrklinke und wenigstens einen Klinkenauslösarm umfaßt.

6. Lanzettenvonichtung nach Anspruch 5, wobei die Sperrklinke Klinkensperrzähne und das Druckende Druckendensperrzähne umfaßt.

7. Lanzettenvorrichtung nach Anspruch 1, wobei der Auslösemechanismus eine Reibungskupplung zum Feststellen des Druckendes umfaßt.

8. Lanzettenvorrichtung nach Anspruch 1, wobei der Auslösemechanismus einen optischen Relaisschalter umfaßt, der dazu eingerichtet ist, eine Zielstellenwulstung einer vorgegebenen Höhe zu detektieren.

9. Lanzettenvorrichtung nach Anspruch 1, wobei der Auslösemechanismus einen elektrischen Relaisschalter umfaßt.

10. Lanzettenvorrichtung nach Anspruch 1, wobei der Auslösemechanismus dazu eingerichtet ist, ein Stechen durch den Lanzettenmechanismus auszulösen, sobald das Drukkende festgestellt worden ist.

## Revendications

1. Lancette comprenant :
un logement ;
un mécanisme de lancement attaché de manière fonctionnelle au logement ;
une pointe de pression destinée à engager un site cible et créer un gonflement de site cible, la pointe de pression étant attachée de manière mobile au logement ; et
**caractérisée par**
un mécanisme déclencheur destiné à détecter un gonflement de site cible d'une hauteur prédéterminée et, ensuite, déclencher une immobilisation de la pointe de pression par rapport au logement, empêchant de ce fait un changement ultérieur de la position du gonflement de site cible par rapport audit logement.

2. Lancette selon la revendication 1, dans laquelle la lancette comprend en outre :
un ressort de poussée destiné à appliquer une force de préchargement contre la pointe de pression.

3. Lancette selon la revendication 2, dans laquelle le ressort de poussée est configuré pour appliquer une force de préchargement comprise dans la plage de 3 N à 13 N contre la pointe de pression.

4. Lancette selon la revendication 2, dans laquelle le ressort de poussée est configuré pour appliquer une force de préchargement comprise dans la plage de 9 N à 10 N contre la pointe de pression.

5. Lancette selon la revendication 1, dans laquelle le mécanisme déclencheur comprend au moins un doigt de verrouillage et au moins un bras déclencheur de doigt.

6. Lancette selon la revendication 5, dans laquelle le doigt de verrouillage comprend des dents et dans laquelle la pointe de pression comprend des dents de roue à rochet de pointe de pression.

7. Lancette selon la revendication 1, dans laquelle le mécanisme déclencheur comprend un embrayage à frottement destiné à immobiliser la pointe de pression.

8. Lancette selon la revendication 1, dans laquelle le mécanisme déclencheur comprend un relais commutateur optique configuré pour détecter un gonflement de site cible d'une hauteur prédéterminée.

9. Lancette selon la revendication 1, dans laquelle le mécanisme déclencheur comprend un commutateur relais électrique.

10. Lancette selon la revendication 1, dans laquelle le mécanisme déclencheur est configuré pour amorcer le lancement par le mécanisme de lancement une fois que la pointe de pression a été immobilisée.
